# EUROPÄISCHE PATENTSCHRIFT

(11) **EP 0 391 846 B1**
(45) Veröffentlichungstag und Bekanntmachung des Hinweises auf die Patenterteilung: **10.03.1993**
(21) Anmeldenummer: 90810234.6
(22) Anmeldetag: 22.03.1990
(51) Int. Cl.: C12M 1/40, C12M 1/12, C12M 1/02

(54) **Biofliessbettreaktor mit Konditionierungsvorrichtung**
Fluidized-bed bioreactor with a conditioning device
Bioréacteur à lit fluidisé conditionné

(30) Priorität: 07.04.1989 CH 1314/89
(43) Veröffentlichungstag der Anmeldung: 10.10.1990
(73) Patentinhaber: GEBRÜDER SULZER AKTIENGESELLSCHAFT, CH-8401 Winterthur (CH)
(72) Erfinder: Birou, Bernard, Dr., CH-8352 Oberscottikon (CH)

(56) Entgegenhaltungen:
- EP-A- 0 185 407
- EP-A- 0 220 591
- EP-A- 0 263 371
- FR-A- 900 839
- FR-A- 2 385 797
- GB-A- 2 093 730
- US-A- 3 114 677
- US-A- 3 969 190
- US-A- 4 446 229

## Beschreibung

Die Erfindung betrifft einen Biofliessbettreaktor, d. h. einen Flüssig-Feststoff-Wirbelschichtreaktor für biologische Systeme, mit Umwälzmitteln, einer Kreislaufrückführung und einer Konditionierungsvorrichtung. Solche Biofliessbettreaktoren sind z. B. aus der PCT-Anmeldung WO 86/05 202, aus FR-A-2 385 797 und EP-A-0 185 407 bekannt. Hier wird ein Teil des Fluids in einer Seitenschlaufe aus dem Reaktorraum herausgeführt, dort in einer externen Konditionierungsvorrichtung bezüglich Temperatur oder zur stofflichen Zusammensetzung verändert und das so behandelte Fluid wieder in den Reaktorraum zurückgeführt. Diese bekannten Biofliessbettreaktoren weisen jedoch noch wesentliche Mängel und Beschränkungen auf. Dies sowohl bezüglich hydrodynamischen als auch der biologischen Reaktionseigenschaften. So sind z. B. Homogenität und Stabilität des Fliessbetts noch mangelhaft, und es treten Rückvermischung und Channeling auf. Infolge hoher Expansionen ist auch die volumetrische Produktivität noch bescheiden. Durch diese ungünstigen hydrodynamischen Bedingungen werden die sehr scherempfindlichen biologischen Zellen beeinträchtigt oder geschädigt. Dadurch wird auch die Qualität der biologischen Reaktionen und der Reaktionsprodukte beeinträchtig. Mit dieser externen, stufenweisen Konditionierung ist es nicht möglich, homogene Bedingungen unter Vermeidung von schädlichen Konzentrations- und Temperaturgradienten zu erreichen. Die Konditionierung und die Durchflussgeschwindigkeit sind bei diesen bekannten Systemen gekoppelt und können daher nicht je separat optimal eingestellt werden. Vor allem ist auch eine Vergrösserung vom Laborfliessbettreaktor auf grössere Produktionseinheiten kaum möglich, oder nur in sehr beschränktem Masse und durch aufwendige mehrstufige Konditionierungsschritte.

Es ist daher Aufgabe der vorliegenden Erfindung, einen Biofliessbettreaktor zu schaffen, welcher diese hydrodynamischen und biologischen Probleme weitgehend überwindet. Der neue Reaktor soll sowohl ein homogenes, stabiles Fliessbett mit geringer Expansion als auch eine optimale, gleichmässige Konditionierung mit minimalen Temperatur- und Konzentrationsgradienten aufweisen. Die Konditionierung und die Strömungsgeschwindigkeit im Reaktionsraum sollen entkoppelt und separat optimierbar sein. Die volumetrische und die biologische Produktivität sollen wesentlich erhöht und ein scale up möglich sein.

Diese Aufgabe wird erfindungsgemäss dadurch gelöst, dass beim Biofliessbettreaktor mindestens eine Konditionierungsvorrichtung im Reaktorraum angeordnete Einbauten aufweist, welche in der Form von statischen Mischern angeordnet sind, dass die Einbauten einen, durch eine Wandung vom Reaktorraum getrennten Innenraum für ein Konditionierungsmittel aufweisen und dass der Innenraum an ein externes Konditionierungssystem angeschlossen ist.

Durch die Ausbildung der Einbauten in der Form von statischen Mischern werden im ganzen Reaktorraum optimale und die Mikroorganismen schonende hydrodynamische Bedingungen geschaffen und durch die Ausbildung als Konditionierungsvorrichtung gleichzeitig eine homogene und optimale Konditionierung ermöglicht. Damit werden geringe Expansionen, mehrfach höhere volumetrische und biologische Produktivitäten und verbesserte Reaktionsqualitäten erreicht.

In den abhängigen Ansprüchen werden vorteilhafte Ausbildungen der Erfindung offenbart. So können durch eine dem Konditionierungssystem zugeordnete Steuerungseinheit und durch im Reaktorraum angeordnete Sensoren optimale Bedingungen ermittelt und auch automatisch eingehalten werden. Durch Anordnung der Einbauten in mehreren übereinanderliegenden Zonen unterschiedlicher Orientierung kann die Homogenisierung des Fliessbetts weiter verbessert werden.

Die Wandungen der Einbauten sind auf das Konditionierungsmittel abgestimmt. Zur Stoffaustauschkonditionierung können die Einbauten eine für den zu tauschenden Stoff permeable Wandung aufweisen. Zur blasenfreien Begasung des Reaktorraums kann der Innenraum mit einer Begasungsquelle als Konditionierungssystem verbunden sein. Zur thermischen Konditionierung des Reaktorraums können die Einbauten eine undurchlässige, aber wärmeleitende Wandung aufweisen, wobei der Innenraum mit einem äusseren Heiz- oder Kühlsystem verbunden ist.

Die Einbauten können einfacherweise rohrförmig, plattenförmig oder als sich kreuzende Stege ausgeführt sein, oder sie können auch aus einem über eine Trägerstruktur abgewickelten Schlauchzug bestehen. Die Einbauelemente weisen vorteilhafterweise einen Winkel von mindestens 10° zur Hauptströmungsrichtung im Reaktorraum auf. Oft kann auch ein Winkel von mindestens 45° besonders günstige, geringe Expansionswerte ergeben.

Es können auch zwei oder mehr voneinander unabhängige Konditionierungsvorrichtungen vorgesehen sein, welche je Einbauten im Reaktorraum, eine Wandung und einen Innenraum mit einem separaten Konditionierungssystem aufweisen. So kann etwa mit einer ersten Konditionierungsvorrichtung dem Reaktorraum ein Konditionierungsmittel (z. B. O₂) zugeführt und mit der zweiten Konditionierungsvorrichtung ein Reaktionsprodukt (z. B. NH₄) aus dem Reaktorraum abgeführt werden. Zusätzlich kann auch noch eine thermische Konditionierungsvorrichtung vorgesehen sein.

Im folgenden wird die Erfindung anhand von Ausführungsbeispielen im Zusammenhang mit den Zeichnungen näher erläutert. Dabei zeigt:
Fig. 1 einen erfindungsgemässen Bioreaktor mit Konditionierungsvorrichtung;
Fig. 2a, 2b schematisch Vermischungswirkung und Anordnung einer Konditionierungsvorrichtung im Reaktorraum;
Fig. 3 ein weiteres Beispiel mit verschiedenen Zonen und mit zwei Konditionierungsvorrichtungen;
Fig. 4 eine Anordnung von Konditonierungseinbauten;
Fig. 5 den Verlauf von Expansionen in Funktion der Durchströmungsgeschwindigkeit;
Fig. 6 Verläufe von Konzentrationen und Temperaturen in Funktion der Höhe im Reaktorraum;
Fig. 7 bis 10 verschiedene Beispiele von Konditionierungseinbauten im Reaktorraum.

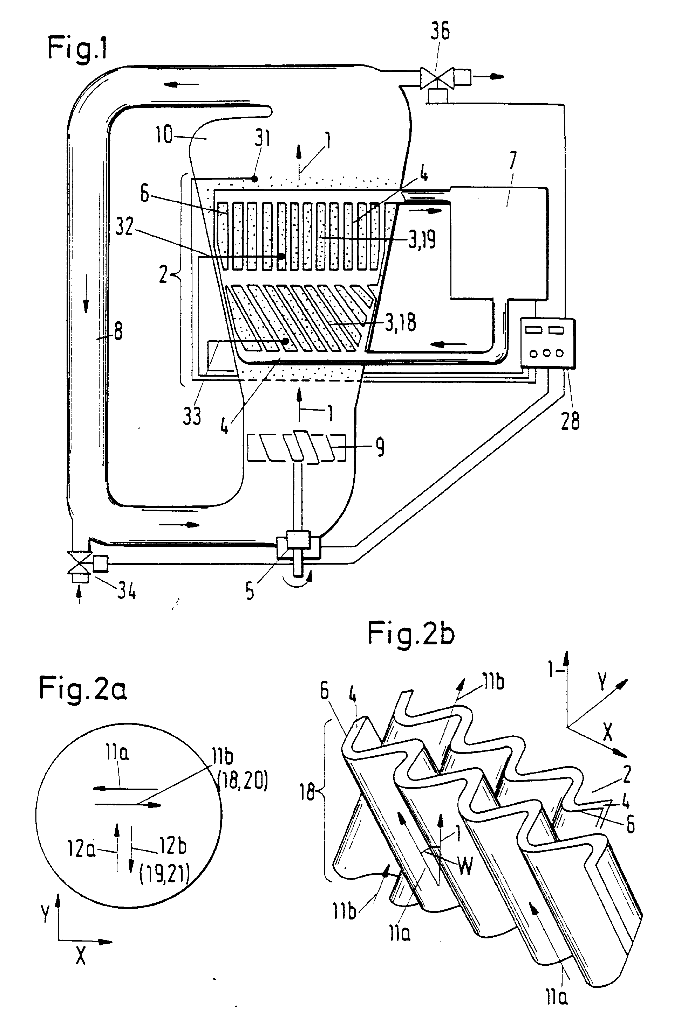

Ein Biofliessbettreaktor in Fig. 1 weist einen Reaktorraum 2, eine Rückflussleitung 8 zum Betreiben im Kreislauf, mit einem Antrieb 5 versehene Umwälzmittel 9 und eine Konditionierungsvorrichtung 3 auf. Am oberen Ausgang des Reaktorraums 2 mit Hauptströmungsrichtung 1 ist eine Ableitung 36 für mindestens einen Teil der Reaktionsprodukte angeordnet und vor dem Eingang des Reaktorraums ermöglicht eine Zuleitung 34 die Zufuhr von Ausgangsstoffen und Trägerflüssigkeit. Sowohl die Zuleitung 34 als auch die Ableitung 36 sind zur Dosierung mit einstellbaren Ventilen versehen, welche mit einer Steuer-/Regeleinheit 28 verbunden und von dieser steuerbar sind. Die Einbauten 3 der Konditionierungsvorrichtung im Reaktorraum 2 bestehen aus einer Vielzahl von Elementen, welche in Form von statischen Mischern ausgebildet bzw. angeordnet sind. Dabei sind die Einbauelemente hohl, d.h. sie weisen einen gemeinsamen Innenraum 4 auf, welcher durch eine Wandung 6 vom Reaktorraum getrennt ist. Zur thermischen Konditionierung weisen die Einbauten eine undurchlässige, wärmeleitende Wandung (6) auf. Der Innenraum 4 ist mit einem externen Konditionierungssystem 7 verbunden, wobei ein Konditionierungsmittel vom externen Konditionierungssystem 7 in den Innenraum 4 der Einbauten im Reaktorraum geführt wird. Diese Konditionierung kann Zufuhr und/oder Abfuhr von verschiedenen Stoffen in den bzw. aus dem Reaktorraum 2 umfassen. Bei thermischer Konditionierung wird ein Heizmittel oder ein Kühlmittel in den Innenraum 4 im Reaktorraum geführt. Die Steuerung des Konditionierungssystems 7 erfolgt durch eine zugeordnete Steuer-/Regeleinheit 28 mit Anzeige- und Registrierelementen. Dabei wird der Einfluss der Konditionierung im Reaktorraum durch Sensoren 31, 32, 33 erfasst und in die Steuereinheit 28 übermittelt. Diese Sensoren messen die für Bioreaktionen wichtigen Grössen wie Konzentrationen verschiedener Stoffe, Temperaturen, pH-Werte und Partialdrücke. Durch entsprechende Anordnung der Sensoren 31 bis 33 kann auch die räumliche Verteilung dieser Messgrössen im Reaktorraum ermittelt werden. Damit können insbesondere die Homogenität der Reaktionen, auftretende Gradienten und allfälliges lokales Ueberschreiten von vorgegebenen Grenzwerten erfasst werden.

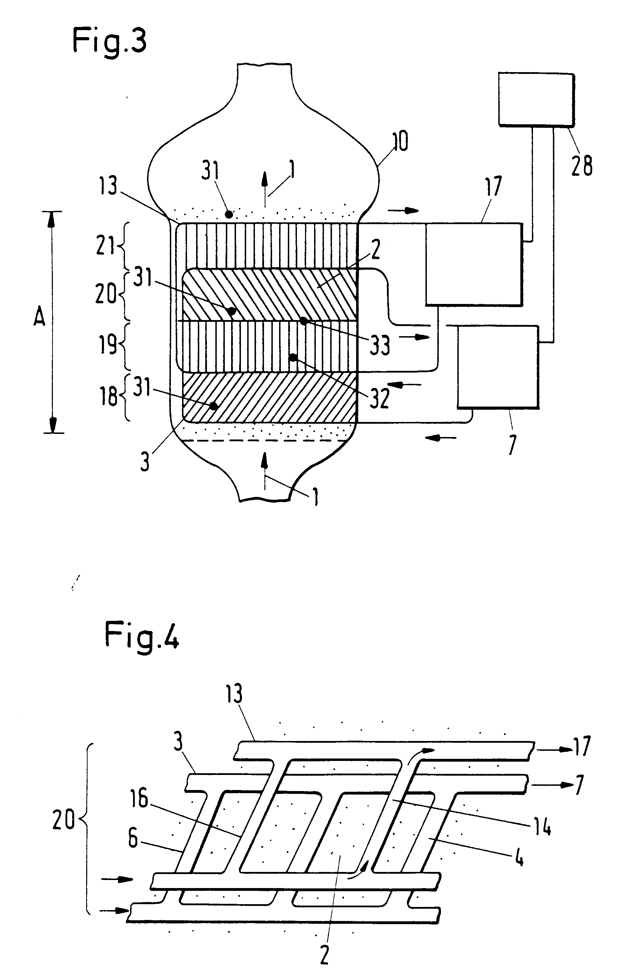

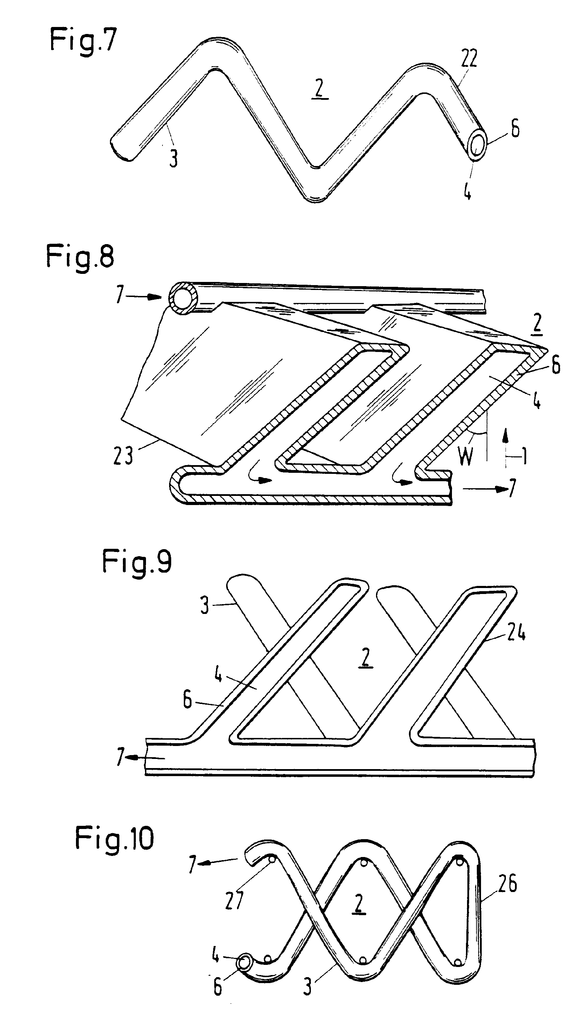

So können z.B. mehrere gleichartige Sensoren 31 für eine bestimmte Konzentrationsmessung auf verschiedenen Höhen im Reaktorraum angeordnet sein, wie in Fig. 3 dargestellt. Eine Anordnung der Einbauten und deren mechanische Misch- und Homogenisierungswirkung bezogen auf die Haupt-Strömungsrichtung 1 wird in den Figuren 2a,b dargestellt. Figur 2a zeigt eine Ansicht in Strömungsrichtung 1. Die beiden übereinanderliegenden Zonen 18, 19 sind dabei in unterschiedlicher Orientierung im Reaktorraum 2 angeordnet. Die Einbauelemente der unteren Zone 18 sind abwechselnd in Querrichtung X, je nach links 11a bzw. nach rechts 11b orientiert. Die Einbauelemente der oberen Zone 19 sind dagegen abwechselnd in dazu senkrechten Richtungen Y, 12a, 12b, orientiert. Alle Elemente weisen dabei zur Hauptströmungsrichtung 1 einen Winkel W von mindestens 10° vorzugsweise oft sogar von mindestens 45° auf (Fig. 2b und 8).

Es können mehr als zwei Zonen unterschiedlicher Orientierung vorgesehen sein. In Fig. 3 und 4 sind es 4 Zonen 18, 19, 20, 21, welche mit zwei externen, separaten Konditionierungssystemen 7 und 17 verbunden sind. Dabei sind die Einbauten 3 mit Innenraum 4, Wandung 6 und einem ersten Konditionierungsmittel mit dem Konditionierungssystem 7 verbunden. Ein zweites Konditionierungsmittel des Koditionierungssystems 17 fliesst durch den Innenraum 14 mit Wandung 16 der Einbauten 13. Die Konditionierungseinbauten 3 sind hier in den Zonen 18, 19, 20 und die Einbauten 13 in den Zonen 19, 20, 21 des Reaktorraums angeordnet. Die beiden unabhängigen Konditionierungsvorrichtungen 3, 4, 6, 7 bzw. 13, 14, 16, 17 werden beide von der gemeinsamen Steuerungseinheit 28 gesteuert und koordiniert. Fig. 4 zeigt in einem Ausschnitt aus Zone 20 von Fig. 3 wie die beiden separaten Konditionierungsvorrichtungen 3, 4, 6 und 13, 14, 16 in der gleichen Zone 20 angeordnet sein können. Dabei kann die erste Konditionierungsvorrichtung ein Stoffaustauschsystem (7) und die zweite konditionierungsvorrichtung ein Wärmeaustauschersystem (17) aufweisen.

Meist ist es vorteilhaft, die Konditionierungseinbauten im wesentlichen über den ganzen Reaktionsraum gleichmässig verteilt anzuordnen.

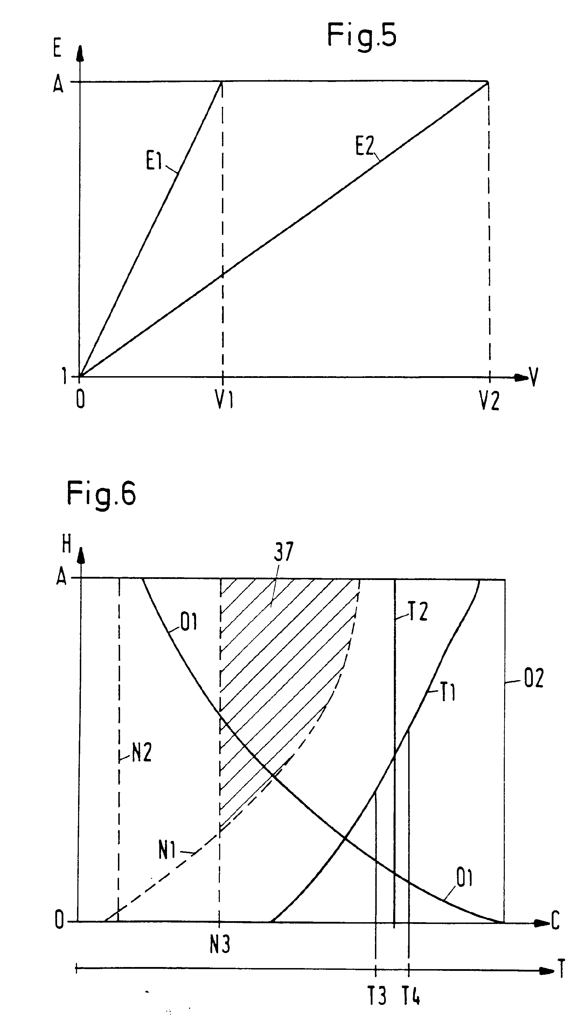

Fig. 5 und 6 illustrieren die besonders vorteilhafte Wirkungsweise des erfingungsgemässen Bioreaktors. In Fig. 5 ist der Verlauf der Expansion E in Funktion der Durchströmungsgeschwindigkeit V dargestellt. Die Expansion E ist dabei definiert als Verhältnis der Fliessbetthöhe bei V>0 zur Fliessbetthöhe bei V = 0. Bei einem bisherigen Bioreaktor mit externer Konditionierung ergibt sich ein mit der Geschwindigkeit V steil ansteigender Verlauf E1 der Expansion. Schon bei einer relativ geringen Geschwindigkeit V1 wird dabei die Reaktorhöhe A erreicht. Die Geschwindigkeit kann hier also höchstens V1 betragen. Mit dem erfindungsgemässen Bioreaktor dagegen wird ein viel flacherer Expansionsverlauf E2 erreicht. Die Strömungsgeschwindigkeit kann dadurch auf den wesentlich höheren Wert V2 gesteigert werden, ohne dass die Expansion die gegebene Reaktorhöhe A übersteigt. Der Betriebsbereich und damit die volumetrische Produktivität des Bioreaktors werden somit massiv erweitert bzw. erhöht. Diese strömungsmechanischen Vorteile können aber erst voll genutzt werden durch die simultane erweiterte biologische Wirksamkeit des Bioreaktors, welche anhand von Konzentrations- und Temperaturprofilen in Fig. 6 erläutert wird. Hier ist der Verlauf von für die Bioreaktionen relevanten Konzentrationen und Temperaturen in Funktion der Höhe H im Reaktorraum dargestellt. Als Beispiele sind dargestellt die O₂ Konzentration O1 (H), die NH₄ Konzentration N1 (H) und die Temperatur T1 (H) für bisherige Bioreaktoren, sowie O2 (H), N2 (H) und T2 (H) für den erfindungsgemässen Bioreaktor. Die für viele Bioreaktionen wichtige Zufuhr von O₂ als Verbrauchsstoff bzw. die Abfuhr von NH₄ als toxischem Reaktionsprodukt zeigt in bisherigen Bioreaktoren eine sehr starke Abhängigkeit von H, d.h. grosse Konzentrationsgradienten. Mit dem erfindungsgemässen Bioreaktor können dagegen weitgehend konstante und optimale Konzentrationprofile O2, N2 über den ganzen Reaktorraum erreicht werden. Der bisherige Verlauf O1 (H) zeigt eine starke Abnahme mit der Höhe, entsprechend nimmt auch die biologische Produktivität im Reaktor ab. Demgegenüber wird O2 (H) auf optimal hohem Niveau konstant gehalten. Umgekehrt nimmt die Konzentration N1 (H) über die Reaktorhöhe ständig zu, während wiederum erfindungsgemäss, N2 (H) auf einem sehr niedrigen Niveau konstant gehalten wird. Sei N3 die für eine bestimmte Bioreaktion maximal zulässige NH₄-Konzentration, so ist also der Betrieb nach bisheriger Kurve N1, welche die Limite N3 schon im unteren Reaktorbereich erreicht und übersteigt, gar nicht zulässig bzw. nicht möglich (schraffierter Bereich 37). N2 (H) dagegen bleibt überall weit unter der Limite N3.

Analoges gilt auch für andere biologisch relevante Grössen, z.B. für pH-Werte und natürlich für die Temperaturen im Reaktorraum. Der bisherige Temperaturverlauf T1 (H) variiert wiederum relativ stark über die ganze Reaktorhöhe A, z.B. von 20° auf 30° C ansteigend. Durch interne Konditionierung (Kühlung) kann dagegen, erfindungsgemäss, ein praktisch konstanter Verlauf T2 erreicht werden (auf weniger als 1° C genau konstant). Damit kann diese Temperatur T2 im ganzen Reaktorraum in einem engen optimalen Bereich zwischen T3 und T4 gehalten werden, was mit den bisherigen extern konditionierten Bioreaktoren jedenfalls nicht möglich ist. Beispielsweise kann T4 - T3 = 1° C betragen.

Die Figuren 7 bis 10 zeigen verschiedene einfache Ausführungen von Konditionierungseinbauten 3 im Reaktorraum 2 mit Innenraum 4, Wandung 6 und Verbindungsleitungen zum Konditionierungssystem 7. Es zeigen
Fig. 7 gebogene rohrförmige Einbauten 22,
Fig. 8 flache, plattenförmige Einbauten 23, welche analog zu den gewellten Platten von Fig. 2b angeordnet sein können und welche hier einen Winkel W von etwa 50° zur Hauptströmungsrichtung 1 bilden,
Fig. 9 sich kreuzende Stege 24 und
Fig. 10 einen auf eine stangenförmige Trägerstruktur 27 abgewickelten flexiblen Schlauchzug 26, welcher sich mit porösen Wänden besonders auch zur schonenden und blasenfreien Begasung eignet.

Zur Illustration der Wirksamkeit des erfindungsgemässen Bioreaktors wird ein einfaches Beispiel angegeben. Eine empfindliche tierische Zellkultur BHK 21 sei gezüchtet auf MEM mit 10 % FCS und mit 150 µm Glaskügelchen als Träger. Dann kann mit dem erfindungsgemässen Biofliessbettreaktor gegenüber bisherigen Reaktoren eine dreimal kleinere Expansion und damit eine dreimal höhere Strömungsgeschwindigkeit und Zellkonzentration von z. B. 3 x 10⁷ Zellen/ml erreicht werden. Auch die volumetrische Produktivität ist damit also dreimal grösser. Hinzu kommt noch die kombinierte biologische Produktivitätserhöhung durch die erfindungsgemässe Konditionierungsvorrichtung. Bei einem spezifischen Sauerstoffbedarf der Zellen von 5 x 10⁻⁶ mg 0₂ / 10⁶ Zellen x Sekunden und einer Fliessgeschwindigkeit von 4 mm/s wird die Sauerstoffkonditionierung schon bei der kleineren Zellkonzentration von 10⁷ Zellen / ml der bisherigen Reaktoren auf einer Reaktorlänge A von 24 cm erschöpft (d. h. von z. B. 60 % auf 10 % O₂-Sättigung reduziert). Mit der erfindungsgemässen Konditionierungsvorrichtung dagegen kann der gewünschte hohe Sauerstoffgehalt von 60 % O₂-Sättigung auch für die höhere Zellenkonzentration von 3 x 10⁷ Zellen / ml über den ganzen Reaktorraum aufrechterhalten werden. Damit ist auch ein scaling up auf wesentlich grössere Reaktoren möglich. Bei eine Konditionierung mit Luft-CO₂ - Gemisch zur Zuführung von O₂ kann auch ein gewünschter optimaler pH-Wert von z. B. 7,2 durch Variation des CO₂-Partialdrucks in der Gasphase in bekannter Weise über den ganzen Reaktorraum eingehalten werden.

## Patentansprüche

1. Biofliessbettreaktor (Flüssig-Feststoff-Wirbelschichtreaktor für biologische Systeme) mit Umwälzmitteln, einer Kreislauf-Rückführung und einer Konditionierungsvorrichtung, dadurch **gekennzeichnet**, dass mindestens eine Konditionierungsvorrichtung im Reaktorraum (2) angeordnete Einbauten (3) aufweist, welche in der Form von statischen Mischern angeordnet sind, dass die Einbauten (3) einen, durch eine Wandung (6) vom Reaktorraum (2) getrennten Innenraum (4) für ein Konditionierungsmittel aufweisen und dass der Innenraum (4) an ein externes Konditionierungssystem (7) angeschlossen ist.

2. Biofliessbettreaktor nach Anspruch 1, dadurch gekennzeichnet, dass dem Konditionierungssystem (7) eine Steuerungseinheit (28) zugeordnet ist und dass im Reaktorraum (2) Sensoren (31, 32, 33) angeordnet sind, welche mit der Steuerungseinheit (28) verbunden sind.

3. Biofliessbettreaktor nach Anspruch 1, dadurch gekennzeichnet, dass die Einbauten in mindestens zwei übereinander liegenden Zonen (18, 19, 20, 21) unterschiedlicher Orientierung angeordnet sind.

4. Biofliessbettreaktor nach Anspruch 1, dadurch gekennzeichnet, dass die Einbauten (3) eine permeable Wandung (6) aufweisen und dass der Innenraum (4) mit einem Stoffaustauschsystem (7) verbunden ist.

5. Biofliessbettreaktor nach Anspruch 4 zur blasenfreien Begasung des Reaktorraums (2), dadurch gekennzeichnet, dass der Innenraum (4) mit einer Begasungsquelle (7) verbunden ist.

6. Biofliessbettreaktor nach Anspruch 1 zur thermischen Konditionierung des Reaktorraums, dadurch gekennzeichnet, dass die Einbauten eine undurchlässige, wärmeleitende Wandung (6) aufweisen, und dass der Innenraum (4) mit einem Heiz- oder Kühlsystem (7) verbunden ist.

7. Biofliessbettreaktor nach Anspruch 1, dadurch gekennzeichnet, dass die Einbauten (3) rohrförmig (22), plattenförmig (23) oder als sich kreuzende Stege (24) ausgeführt sind.

8. Biofliessbettreaktor nach Anspruch 1, dadurch gekennzeichnet, dass die Einbauten aus einem über eine Trägerstruktur (27) abgewickelten Schlauchzug (26) bestehen.

9. Biofliessbettreaktor nach Anspruch 1, dadurch gekennzeichnet, dass die Einbauelemente (6) einen Winkel von mindestens 10° zur Hauptströmungsrichtung (1) im Reaktorraum aufweisen.

10. Biofliessbettreaktor nach Anspruch 1, dadurch gekennzeichnet, dass die Einbauelemente (6) einen Winkel von mindestens 45° zur Hauptströmungsrichtung (1) im Reaktorraum aufweisen.

11. Biofliessbettreaktor nach Anspruch 1, dadurch gekennzeichnet, dass mindestens zwei voneinander unabhängige Konditionierungsvorrichtungen vorgesehen sind, welche je Einbauten (3, 13) im Reaktorraum (2), eine Wandung (6, 16) und einen Innenraum (4, 14), welcher mit einem Konditionierungssystem (7, 17) verbunden ist, aufweisen.

12. Biofliessbettreaktor nach Anspruch 11, dadurch gekennzeichnet, dass mit der ersten Konditionierungsvorrichtung (3, 4, 6, 7) dem Reaktorraum ein Konditionierungsstoff zugeführt und mit der zweiten Konditionierungsvorrichtung (13, 14, 16, 17) ein Reaktionsprodukt aus dem Reaktorraum (2) abgeführt wird.

13. Biofliessbettreaktor nach Anspruch 11, dadurch gekennzeichnet, dass die erste Konditionierungsvorrichtung ein Stofftauschsystem (7) und die zweite Konditionierungsvorrichtung ein Wärmetauschsystem (17) aufweist.

## Claims

1. A fluidised-bed bio-reactor (liquid-solid fluidised bed reactor for biological systems) comprising circulating means, recycling means and a conditioning device, characterised in that at least one conditioning device has packings (3) disposed in the reactor chamber (2), said packings being arranged in the form of static mixers, in that the packings (3) have an interior (4) - separated from the reactor chamber (2) by a wall (6) - for a conditioning medium and in that the interior (4) is connected to an external conditioning system (7).

2. A fluidised-bed bio-reactor according to claim 1, characterised in that a control unit (28) is associated with the conditioning system (7) and in that sensors (31, 32, 33) are disposed in the reactor chamber (2) and are connected to the control unit (28).

3. A fluidised-bed bio-reactor according to claim 1, characterised in that the packings are disposed in at least two superposed zones (18, 19, 20, 21) of different orientations.

4. A fluidised-bed bio-reactor according to claim 1, characterised in that the packings (3) have a permeable wall (6) and in that the interior (4) is connected to a mass-transfer system (7).

5. A fluidised-bed bio-reactor according to claim 4 for bubble-free gasification of the reactor chamber (2), characterised in that the interior (4) is connected to a gasification source (7).

6. A fluidised-bed bio-reactor according to claim 1 for thermal conditioning of the reactor chamber, characterised in that the packings have an impermeable thermally conductive wall (6) and in that the interior (4) is connected to a heating or cooling system (7).

7. A fluidised-bed bio-reactor according to claim 1, characterised in that the packings (3) are tubular (22), plate-shaped (23) or in the form of crossing webs (24).

8. A fluidised-bed bio-reactor according to claim 1, characterised in that the packings consist of a length of flexible tubing (26) unwound over a supporting structure (27).

9. A fluidised-bed bio-reactor according to claim 1, characterised in that the packings (6) have an angle of at least 10° to the main direction of flow (1) in the reactor chamber.

10. A fluidised-bed bio-reactor according to claim 1, characterised in that the packings (6) have an angle of at least 45° to the main direction of flow (1) in the reactor chamber.

11. A fluidised-bed bio-reactoraccording to claim 1, characterised in that at least two independent conditioning devices are provided each having packings (3, 13) in the reactor chamber (2), a wall (6, 16) and an interior (4, 14) connected to a conditioning system (7, 17).

12. A fluidised-bed bio-reactor according to claim 11, characterised in that a conditioning medium is fed to the reactor chamber by the first conditioning device (3, 4, 6, 7) and a reaction product is discharged from the reactor chamber (2) by the second conditioning device (13, 14, 16, 17).

13. A fluidised-bed bio-reactor according to claim 11, characterised in that the first conditioning device comprises a mass-transfer system (7) and the second conditioning device comprises a heat-exchange system (17).

## Revendications

1. Bioréacteur à lit fluidisé (réacteur à couches tourbillonnaires de liquide et de solide pour systèmes biologiques) comprenant des organes d'entretien d'une circulation, un conduit de recyclage en circuit fermé et un dispositif de conditionnement, caractérisé en ce qu'au moins un dispositif de conditionnement comprend des éléments rapportés (3) disposés dans la chambre (2) du réacteur et agencés en forme de mélangeurs statiques, en ce que les éléments rapportés (3) comprennent un espace interne (4) destiné à un agent de conditionnement et séparé par une cloison (6) de la chambre (2) du réacteur et en ce que l'espace interne (4) est raccordé à un système externe de conditionnement (7).

2. Bioréacteur à lit fluidisé selon la revendication 1, caractérisé en ce qu'un groupe de commande (28) est affecté au système de conditionnement (7) et en ce que des détecteurs (31, 32, 33) reliés au groupe de commande (28) sont disposés dans la chambre (2) du réacteur.

3. Bioréacteur à lit fluidisé selon la revendication 1, caractérisé en ce que les éléments rapportés sont disposés dans au moins deux zones superposées (18, 19, 20, 21) dont les orientations sont différentes.

4. Bioréacteur à lit fluidisé selon la revendication 1, caractérisé en ce que les éléments rapportés (3) comprennent une cloison perméable (6) et en ce que l'espace interne (4) est relié à un système d'échange de matières (7).

5. Bioréacteur à lit fluidisé selon la revendication 4, destiné à un balayage de la chambre du réacteur (2) par un gaz sans bulles, caractérisé en ce que l'espace interne (4) est relié à une source de gaz de balayage (7).

6. Bioréacteur à lit fluidisé selon la revendication 1, pour le conditionnement thermique de la chambre du réacteur, caractérisé en ce que les éléments rapportés comportent une cloison imperméable (6) conductrice de la chaleur et en ce que l'espace interne (4) est relié à un système (7) de chauffage ou de refroidissement.

7. Bioréacteur à lit fluidisé selon la revendication 1, caractérisé en ce que les éléments rapportés (3) sont tubulaires (22), en forme de plaques (23) ou constitués de barreaux (24) qui se croisent.

8. Bioréacteur à lit fluidisé selon la revendication 1, caractérisé en ce que les éléments rapportés consistent en une certaine longueur de tuyau flexible (26) déployé sur une structure de support (27).

9. Bioréacteur à lit fluidisé selon la revendication 1, caractérisé en ce que les éléments rapportés (6) inscrivent un angle d'au moins 10° avec la direction principale (1) de circulation dans la chambre du réacteur.

10. Bioréacteur à lit fluidisé selon la revendication 1, caractérisé en ce que les éléments rapportés (6) inscrivent un angle d'au moins 45° avec la direction principale (1) de circulation dans la chambre du réacteur.

11. Bioréacteur à lit fluidisé selon la revendication 1, caractérisé en ce qu'au moins deux dispositifs de conditionnement indépendants l'un de l'autre sont prévus et chacun d'eux comprend des éléments rapportés (3, 13) dans la chambre du réacteur (2), une cloison (6, 16) et un espace interne (4, 14) qui est relié à un système de conditionnement (7, 17).

12. Bioréacteur à lit fluidisé selon la revendication 11, caractérisé en ce que le premier dispositif de conditionnement (3, 4, 6, 7) envoie dans la chambre du réacteur une substance de conditionnement et le second dispositif de conditionnement (13, 14, 16, 17) évacue de la chambre (2) du réacteur un produit de réaction.

13. Bioréacteur à lit fluidisé selon la revendication 11, caractérisé en ce que le premier dispositif de conditionnement comprend un système d'échange de matières (7) et le second dispositif de conditionnement comprend un système (17) d'échange de chaleur.
